# EUROPEAN PATENT APPLICATION

(11) **EP 3 323 441 A1**
(43) Date of publication of application: **23.05.2018**
(21) Application number: 16836636.7
(22) Date of filing: 15.08.2016
(51) Int. Cl.: A61L 27/56, A61L 27/04, A61L 27/02, C22C 1/08, C22C 1/05

(54) **HIERARCHICAL POROUS MATERIAL**

(30) Priority: 19.08.2015 CN 201510510836
(71) Applicant: Chongqing Runze Pharmaceutical Co., Ltd., Chongqing 400042 (CN)
(72) Inventor: YE, Lei, Chongqing 400042 (CN)
(74) Representative: Inal, Aysegul Seda
(86) International application number: PCT/CN2016/095329
(87) International publication number: WO 2017/028772

(57) **Abstract**

A hierarchical porous material consists of multistage porous materials, comprising a material body, the body is formed by a pore cavity graded according to the pore size of the material and a cavity wall surrounding to form the pore cavity. The pore cavities are uniformly distributed. The characteristics are as follows. A lower-level pore cavities are disposed on the cavity wall of an upper-level pore cavity formed by surrounding a three-dimensional space. Each level of pore cavities are mutually connected and the pore cavities within same level are also connected with each other. The uniform distribution of the pore cavities means that similar amount of pore cavities are distributed under any unit volume of the hierarchical porous material. The hierarchical structure of the cavity of the material enables it to meet a wide range of functional requirements. The uniformity of the distribution of pores of hierarchical porous materials is measured at a small unit volume scale, and the hierarchical porous material is highly uniform, thus ensuring the uniformity of various properties of the hierarchical porous material.

## Description

### Technical Field

The present invention relates to a porous material, particularly to a hierarchical porous material.

### Background of the Invention

Porous material has the advantages of low relative density, high specific strength, high specific surface area, light weight, soundproofing, thermal insulation and good permeability, which contributes to a wide application in fields of spaceflight, aviation, chemical industry, building materials, metallurgy, atomic energy, petrification, machinery, medicine, environmental protection, etc. In porous material, hierarchical porous material has attracted more and more attentions due to its unique properties. Hierarchical porous materials not only have the advantages of each level of the porous material at the same time, but also have the advantages that a certain single-pore porous material does not have. For example, microporous-mesoporous materials have the advantages of both the microporous materials and the mesoporous materials, making it superior to a single-pore porous material. Hierarchical porous carbon materials respectively with pores of 317 nm and mesoporous of 10 nm are used as electrodes in catalytic reaction, catalytic activity of which is significantly higher than that of conventional catalysts. A three-dimensionally ordered macroporous-mesoporous-microporous molecular sieve, combining the advantages of hierarchical pore structure with that of molecular sieve nanocrystals, gives a more open three-dimensional pore structure of the molecular sieve. Meanwhile, the molecular sieve nanocrystals greatly shorten the material transport path, thus effectively improving the catalytic activity of the resulting molecular sieve. Silicon dioxide (SiO₂) with hierarchical mesoporous-macroporous structure can be used as immobilized biomolecules, such as lysozyme-immobilized guest material with high adsorption rate and high enzyme loading. Titanium dioxide (TiO₂), zirconium dioxide (ZrO₂) and the composite of titanium dioxide-zirconium dioxide (TiO₂-ZrO₂) with mesoporous-macroporous structure can be used as a carrier loaded with palladium (Pd) catalyst, the prepared catalyst shows excellent performance in the catalytic oxidation of toluene and chlorobenzene. Manganese oxide with hierarchical porous structure can be used as water treatment agent, performing a selective absorption to methyl blue and Hg²⁺ under the near-neutral condition.

At present, hierarchical porous materials mentioned in the general literature refer to materials having at least two combinations of micropores (pore size<2 nm), mesoporesous (2 nm<pore size<50 nm) and macropores (pore size>50 nm). With the increasing demands, the need for hierarchical porous materials is not limited within the categories defined above. For example, sometimes nanoscale-micron secondary porous materials are needed, and sometimes nanoscale-dozens of micron-hundreds of micron tertiary porous materials are needed, which has different requirements on the pore's structure, connectivity, etc. The existing and mature porous material lacks this hierarchical porous material.

A method of preparing biomimetic artificial bone with hierarchical (micron/nano) pore structure is provided by patent No: CN201210185031, introducing a method achieved as follows. First, a 150-800 µm gradient through-hole is formed by sintering, using a selective laser (spot diameter is in micron scale); next, a 10-100 µm random spherical pore is formed by the oxidation and decomposition of a small amount of mixed polymer microspheres in the sintering process; finally, a dozen of nanometer pores with irregular surface are obtained through the corrosion process. With this method, a three-dimensional hierarchical pore structure is finally obtained. Due to the randomness and irregularity of the pore structure, regular pores cannot be prepared by this method. The hierarchical porous material with this structure still cannot meet the functional requirements of the biomimetic artificial bone.

In addition, the demand for applying many hierarchical porous materials provides uniformity thereof, i.e. the pore size and distribution are uniform, thus making uniform performance. However, in fact, a large number of hierarchical porous materials do not satisfy the requirement, without sufficient uniformity. Although some materials assert achieving a higher uniformity, which is still the uniformity at a large volume scale. If compared under a small volume scale, for example, a plurality of three-dimensional blocks with volumes less than or equal to one cubic centimeter are randomly selected to measure the quality, the difference of non-uniform degree is still very large, resulting in the non-uniformity in the properties of hierarchical porous materials such as strength, elasticity modulus, etc., thereby seriously affects the function.

### Summary of the Invention

The object of the present invent is to provide a hierarchical porous material with suitable structure, controllability and high uniformity.

The object of the present invention is achieved by the following technical solution.

A hierarchical porous material consisting of multilevel porous materials, includes a material body. The body is formed by pore cavities classified into different levels according to the pore size of the material and a cavity wall surrounding to form the pore cavity. The pore cavities are uniformly distributed and a lower-level of pore cavities are disposed on the cavity wall of an upper-level pore cavity formed by surrounding a three-dimensional space of the upper-level pore cavities. Pore cavities at all levels are mutually connected and pore cavities within each level are also connected with each other. The distribution of the pore cavities is such that pore cavities are uniformly distributed under any unit-level volume of the hierarchical porous material.

More specifically, in the body of the hierarchical porous material, each level porous material of the multilevel porous material is a continuous structure, so that each level porous material can exist as an independent porous material in the body to exert its own function.

Further, each level porous material in the material body is a continuous structure, the maximum outer boundary of each level porous material is equivalent to the space boundary of the entire material body. That is, each level porous material can exist as an independent porous material in the body, and has its own physicochemical properties, which makes the physicochemical properties of the porous materials at all levels different from each other. Having different physicochemical properties in a relatively fixed entire space of material can better meet various functional requirements.

More specifically, in the body of the above-mentioned hierarchical porous material, each level of porous material has its own physicochemical properties, so that each level of porous material can respectively achieve its own unique function, thus the whole material can satisfy various functional requirements.

More specifically, the lower-level porous material forms the cavity wall of the upper-level pore cavity, so that the pore cavity can be hierarchically layered and the structure and pore size of the pore cavity can be reasonably arranged.

More specifically, the cavity wall of the upper-level pore cavity of the above-mentioned hierarchical porous material is composed of a plurality of levels of the lower-level multilevel porous material or all levels of the lower-level porous material, so that the material can meet specific functional requirements.

More specifically, the unit-level volume refers to a cubic centimeter level or a cubic millimeter level or a less unit-level volume.

More specifically, the uniform distribution of the pore cavity means that masses of a plurality of three-dimensional blocks, with volumes less than or equal to one cubic centimeter, and having the same size are substantially the same, the plurality of three-dimensional blocks are randomly selected from hierarchical porous material.

More specifically, the masses are substantially the same means, a plurality of three-dimensional blocks with volumes less than or equal to one cubic centimeter and having the same size are randomly selected from hierarchical porous material, each three-dimensional block is measured to obtain an average value of the mass, and the absolute value of the deviation of the mass of any three-dimensional block from the mass average value is less than or equal to 4% of the mass average value of the three-dimensional blocks.

Further, the masses of a plurality of three-dimensional blocks with volumes less than or equal to one cubic millimeter and the same sizes selected randomly on hierarchical porous material are substantially the same.

More specifically, the masses are substantially the same means, a plurality of three-dimensional blocks with volumes less than or equal to one cubic millimeter and the same sizes are randomly selected from hierarchical porous material, each three-dimensional block is measured to obtain an average value of the mass, and the absolute value of the deviation of the mass of any three-dimensional block from the mass average value is less than or equal to 4% of the mass average value of the three-dimensional blocks.

The advantages of the present invention are as follows.
(1) The present invention provides a porous material having a hierarchical pore structure, the structure of which is clearly defined, and the hierarchical structure of the pore cavity enables it to satisfy various functional requirements.
(2) The present invention provides a specific and definite measurement method for the uniform distribution of the pore cavities of the hierarchical porous material. A measurement of the distribution uniformity of pores of the hierarchical porous material at a small unit-level volume scale is clearly defined. Such a hierarchical porous material is highly uniform, thus ensuring the uniformity of the various properties of the hierarchical porous material.
(3) The hierarchical porous material is three-dimensionally interconnected, including the three-dimensional connection of pores of each level. Pores of each level are three-dimensionally interconnected with good continuity, thereby can fully meet the functional requirements of the material.

### Detailed Description of the Invention

The specific embodiments are given on the premise of the technical solutions of the present invention, but the protection scope of the present invention is not limited to the following embodiments. It will be apparent to those skilled in the art that various changes and modifications may be made without departing from or changing the above technical conception of the present invention, and should be construed as being included in the scope of the present invention.

The embodiments of the present invention are given below in detail.

### Embodiment 1

The hierarchical porous material of the present embodiment is porous alumina with a secondary pore structure. Wherein a uniformly distributed and interconnected second pore cavity is disposed on the cavity wall of a uniformly distributed and interconnected first pore cavity. The first pore and the second pore are also interconnected, which is a three-dimensional interconnection. Each level porous material of the hierarchical porous material is a continuous structure, the maximum outer boundary of each level porous material is equivalent to the space boundary of the entire material body, each level porous material has its own physicochemical properties. The total effective porosity is 60%, the average pore size of the large pores is 10µm, and penetrating small pores with an average pore size of 750nm are on the cavity wall of the large pores.

Nine of three-dimensional blocks having the same size of 10mm×10mm×10mm are randomly selected from the hierarchical porous material by a machining method, the masses of which are measured respectively at a room temperature of 20°C using a METTLER-TOLEDO XP26 Microbalance. Measuring steps are as follows:
1) preheating: turn on the power, preheating the microbalance to a specified time;
2) selecting the basic mode of balance: tap the ON button, turn on the display, and choose the "normal" mode;
3) calibration: using Target (TAR) key to clear, and using Calibration (CAL) minus and calibration weight to calibrate;
4) weighing: pressing the TAR key to display zero, putting the three-dimensional blocks successively on the scale pan, reading the mass value of the three-dimensional block until the figure is stable, i.e. the "0" at the lower left corner of the display disappears.

The measurement results are shown in Table 1, wherein the absolute value of the deviation from the average value is expressed as a percentage, and the value thereof is the absolute value of the deviation from the average value divided by the mass average value. As shown in Table 1, the mass deviation is less than or equal to 4%.

**Table 1**

| No | Mass (mg) | Absolute value of the deviation from the average value (%) |
|---|---|---|
| 1 | 1560.624 | 1.6% |
| 2 | 1568.554 | 1.1% |
| 3 | 1538.420 | 3% |
| 4 | 1552.694 | 2.1% |
| 5 | 1559.038 | 1.7% |
| 6 | 1649.440 | 4% |
| 7 | 1628.822 | 2.7% |
| 8 | 1606.618 | 1.3% |
| 9 | 1609.790 | 1.5% |
| Mass average value | 1586 | |

The preparation method of this material is as follows:
(1) material preparation
   The alumina powder with a particle size of 3µm and urea with a particle size of 860 ± 30 nm are used as pore-forming agent for the smallest pore cavity of the hierarchical porous material. The pore-forming agent is mixed uniformly and the starch with a particle size of 860 ± 30 nm is used as binder. A slurry is prepared by the alumina powder, urea, starch and distilled water with the volume ratio of 3:1.5:1:12.
   The slurry is uniformly filled into a polyester foam with a strut diameter of 15 µm by a foam impregnation method to form a green body and dry the green body, and then crush to obtain mixed grains with a grain size of 15 µm containing a raw material, a pore-forming agent and a polyester foam.
(2) The mixed grains and methyl cellulose with a particle size of 15 µm are uniformly mixed into a closed mould by volume ratio of 3: 1 to be pressed into a compact green body.
(3) The compact green body is vacuum sintered. After the sintering, the green body is subjected to conventional follow-up treatment according to the alumina process to obtain porous alumina with secondary pores.

The material can be used for solid-liquid separation, achieving accurate grading filtration. Pores of two levels respectively filter particles with different sizes, avoiding the accumulation of particles and achieving the efficient separation.

### Embodiment 2

The hierarchical porous material of the present embodiment is porous nickel with a secondary pore structure. The hierarchical porous material is classified into different levels according to the pore size of the material. Wherein all levels of pores are three-dimensionally interconnected. Each level porous material of the hierarchical porous material is a continuous structure, the maximum outer boundary of each level porous material is equivalent to the space boundary of the entire material body, each level porous material has its own physicochemical properties. The total effective porosity is 70%, the average pore size of the large pores is 30 µm, and a penetrating small pore with an average pore size of 600 nm is on the cavity wall of the large pores.

Nine of three-dimensional blocks having the same size of 1mm×1mm×1mm are randomly selected from the hierarchical porous material by a machining method, the masses of which are measured respectively using a METTLER-TOLEDO XP26 Microbalance. Measuring temperature and steps are the same as that in embodiment 1. The results are shown in Table 2, wherein the absolute value of the deviation from the average value is expressed as a percentage, and the value thereof is the absolute value of the deviation from the average value divided by the mass average value. As shown in Table 2, the mass deviation is less than or equal to 4%.

**Table 2**

| No | Mass (mg) | Absolute value of the deviation from the average value (%) |
|---|---|---|
| 1 | 2.635 | 1.3% |
| 2 | 2.638 | 1.2% |
| 3 | 2.587 | 3.1% |
| 4 | 2.606 | 2.4% |
| 5 | 2.622 | 1.8% |
| 6 | 2.774 | 3.9% |
| 7 | 2.742 | 2.7% |
| 8 | 2.707 | 1.4% |
| 9 | 2.723 | 2% |
| Mass average value | 2.670 | |

The preparation method of the porous nickel is as follows:
(1) material preparation
   Nickel powder with a particle size of 3µm and ammonium chloride with a particle size of 700 nm are used as pore-forming agents for the smallest pore. The pore-forming agents are mixed uniformly and the starch with a particle size of 700 nm is used as binder. A slurry is prepared by the nickel powder, ammonium chloride, starch and distilled water with the volume ratio of 2:1.5:1:10.
   The slurry is uniformly filled into a polyester foam with a strut diameter of 40 µm by a foam impregnation method to form a green body and dry the green body, and then crush to obtain a mixed grains with a grain size of 40 µm containing a nickel powder, a pore-forming agent and a polyester foam.
(2) The mixed grains and ethyl cellulose with a particle size of 40 µm are uniformly mixed into a closed mould by volume ratio of 3: 1 to be pressed into a compact green body.
(3) The compact green body is vacuum sintered. After the sintering, the embryoid body is subjected to conventional follow-up treatment according to the alumina process to obtain porous nickel with secondary pores.

This kind of material is used to make electrodes, which has the advantages of low energy consumption and high efficiency compared with the traditional single-cavity porous materials.

### Embodiment 3

The hierarchical porous material of the present embodiment is porous tantalum with a tertiary pore structure. Wherein a uniformly distributed and interconnected secondary pore cavities are disposed on the cavity wall of a uniformly distributed and interconnected first pore cavity, a uniformly distributed and interconnected tertiary pore cavities are disposed on the cavity wall of the uniformly distributed and interconnected secondary pore cavity. The first, secondary and tertiary pore cavities are also interconnected, which is a three-dimensional interconnection. Each level porous material is a continuous structure, the maximum outer boundary of each level porous material is equivalent to the space boundary of the entire material body, each level porous material of the hierarchical porous material has its own physicochemical properties. The total effective porosity is 80%, the average pore size of the first pores is 510 µm, the penetrating second pores with an average pore size of 25 µm are disposed on the cavity wall of the first pore, and the penetrating third pores with an average pore size of 780 nm are disposed on the cavity wall of the secondary pore.

Nine of three-dimensional blocks having the same size of 10 mm×10 mm×10 mm are randomly selected from the hierarchical porous material by a machining method, the masses of which are measured respectively using a METTLER-TOLEDO XP26 Microbalance. Measuring temperature and steps are the same as that in embodiment 1. The results are shown in Table 3, wherein the absolute value of the deviation from the average value is expressed as a percentage, and the value thereof is the absolute value of the deviation from the average value divided by the mass average value. As shown in Table 3, the mass deviation is less than or equal to 4%.

**Table 3**

| No | Mass (mg) | Absolute value of the deviation from the average value (%) |
|---|---|---|
| 1 | 3282.624 | 1.6% |
| 2 | 3292.63 | 1.3% |
| 3 | 3242.592 | 2.8% |
| 4 | 3265.944 | 2.1% |
| 5 | 3285.960 | 1.5% |
| 6 | 3469.440 | 4% |
| 7 | 3409.392 | 2.2% |
| 8 | 3396.048 | 1.8% |
| 9 | 3379.368 | 1.3% |
| Mass average value | 3336 | |

The preparation method of this material is as follows:
(1) material preparation
   Polystyrene beads with a particle size of 900 ± 30 nm are selected to assemble to form a three-dimensional ordered colloid template. Tantalum nanocrystal solution is prepared, introducing the tantalum nanocrystal solution into the three-dimensional colloid template made of polystyrene beads, drying the mixture of the three-dimensional colloid template / tantalum nanocrystal solution and then breaking it into grains with a grain size of 5 µm.
(2) Starch with a particle size of 900 ± 30 nm is selected to mix with distilled water by a weight ratio of 1:40 to prepare starch solution. A slurry is prepared by the above particles, ethyl cellulose with a particle size of 35 µm and the starch solution by the weight ratio of 12: 1: 8, which is uniformly impregnated into a polyester foam with a pore size of 600 ± 20 µm.
(3) The impregnated polyester foam is sintered in a vacuum or a protective atmosphere and then subjected to conventional follow-up treatment according to the tantalum process to obtain porous tantalum with tertiary pores.

The porous tantalum can be used as a bone regeneration material. The size of the first pore is particularly suitable for the growth of living tissues such as blood vessels. The secondary pore is particularly suitable for the residence of various kinds of cells. The tertiary pore is particularly favorable for satisfying the needs of cell adhesion and differentiation due to the large number of nanopores. It also has a high specific surface area and can load a large amount of growth factors. Moreover, the connectivity of the pores is good with the pores at all levels interconnected and the pores within each level connected with each other, which can fully meet the infiltration and transmission of blood and tissue fluid, achieving the excretion of the products of protein degradation and metabolites. Therefore, it is a real bone regeneration material.

### Embodiment 4

The hierarchical porous material of the present embodiment is porous titanium with a tertiary pore structure. Wherein a uniformly distributed and interconnected secondary pore cavity is disposed on the cavity wall of a uniformly distributed and interconnected first pore cavity, a uniformly distributed and interconnected tertiary pore cavity is disposed on the cavity wall of the uniformly distributed and interconnected secondary pore cavity. The first, secondary and tertiary pore cavities are also interconnected, which is a three-dimensional interconnection. Each level porous material is a continuous structure, the maximum outer boundary of each level of porous material is equivalent to the space boundary of the entire material body, each level porous material has its own physicochemical properties. The total effective porosity is 74%, the average pore size of the first pores is 450 µm, the penetrating second pores with an average pore size of 30 µm are disposed on the cavity wall of the first pore, and the penetrating third pores with an average pore size of 670 nm are disposed on the cavity wall of the second pore.

Nine of the three-dimensional blocks having the same size of 10 mm×10 mm×10 mm are randomly selected from the hierarchical porous material by a machining method, the masses of which are measured respectively using a METTLER-TOLEDO XP26 Microbalance. Measuring temperature and steps are the same as that in embodiment 1. The results are shown in Table 4, wherein the absolute value of the deviation from the average value is expressed as a percentage, and the value thereof is the absolute value of the deviation from the average value divided by the mass average value. As shown in Table 4, the mass deviation is less than or equal to 4%.

**Table 4**

| No | Mass (mg) | Absolute value of the deviation from the average value (%) |
|---|---|---|
| 1 | 1156.184 | 1.4% |
| 2 | 1135.077 | 3.2% |
| 3 | 1151.493 | 1.8% |
| 4 | 1157.356 | 1.3% |
| 5 | 1145.630 | 2.3% |
| 6 | 1217.159 | 3.8% |
| 7 | 1197.225 | 2.1% |
| 8 | 1191.362 | 1.6% |
| 9 | 1201.915 | 2.5% |
| Mass average value | 1172.600 | |

The preparation method of the porous titanium is as follows:
(1) material preparation
   The titanium powder with a particle size of 2µm is used as a raw material, starch with a particle size of 770 nm is used as pore-forming agent for the smallest pore cavity, and stearic acid with a particle size of 770 nm is used as binder. A slurry is prepared by the titanium powder, starch, stearic acid and distilled water with the volume ratio of 3:1:1:11.
   The slurry is uniformly filled into a polyester foam with a strut diameter of 30 µm by a foam impregnation method to form a green body and dry the green body, and then crush to obtain a mixed grains with a grain size of 30 µm containing a raw material, a pore-forming agent and a polyester foam.
(2) The mixed grains and methyl cellulose with a particle size of 30 µm are uniformly mixed in a volume ratio of 3:1 to pour into a three-dimensionally interconnected polyester foam with a strut diameter of 560±20 µm and a pore size of 400±15 µm, then putting the polyester foam to a closed mould to press into a compact green body.
(3) The compact green body is vacuum sintered. After the sintering, the green body is subjected to conventional follow-up treatment according to the titanium process to obtain porous titanium with tertiary pores.

Similar to embodiment 3, this material is particularly suitable for using as bone regeneration material.

## Claims

1. A hierarchical porous material consists of a multilevel porous material, comprising:
a material body, the material body is formed by a pore cavity classified according to a pore size of the multilevel porous material; and
a cavity wall surrounding to form the pore cavity;
wherein
the pore cavities are uniformly distributed, and a lower-level of pore cavities are disposed on the cavity wall of an upper-level pore cavity formed by surrounding a three-dimensional space;
the pore cavities of a same level are mutually connected, the pore cavities of different levels are mutually connected; and
a uniform distribution of the pore cavity means that similar amount of pore cavities are distributed over any unit-level volume of the hierarchical porous materials.

2. The hierarchical porous material of claim 1, wherein each level of the multilevel porous material in the material body is a continuous structure.

3. The hierarchical porous material of claim 2, wherein a maximum outer boundary of each level of the multilevel porous material is equivalent to a space boundary of the entire material body.

4. The hierarchical porous material of claim 1, 2 or 3, wherein each of the plurality level of the multilevel porous material in the material body has its own physicochemical properties.

5. The hierarchical porous material of any one of claims 1-4, wherein a lower-level porous material forms the cavity wall of the upper-level pore cavity.

6. The hierarchical porous material of any one of claims 1-4, wherein, the cavity wall of the upper-level pore is formed by a plurality of lower-level multilevel porous materials.

7. The hierarchical porous material of any one of claims 1-4, wherein the cavity wall of the upper-level pore is formed by a composite of all levels of the lower-level porous material.

8. The hierarchical porous material of any one of claims 1-7, wherein a unit-level volume refers to a cubic centimeter level or cubic millimeter level or a less unit-level volume.

9. The hierarchical porous material of claim 8, wherein the uniform distribution of the pore cavity means that masses of a plurality of three-dimensional blocks with volumes less than or equal to one cubic centimeter and the same sizes randomly selected from the hierarchical porous material are substantially the same.

10. The hierarchical porous material of claim 9, wherein the masses are substantially the same means, the plurality of three-dimensional blocks with volumes less than or equal to one cubic centimeter and having the same size are randomly selected from the hierarchical porous material, each of the plurality of three-dimensional block is measured to obtain an average value of the mass, and an absolute value of a deviation of the mass of any of the plurality of three-dimensional blocks from the mass average value is less than or equal to 4% of the mass average value of the plurality of three-dimensional blocks.

11. The hierarchical porous material of claim 8, wherein the masses of a plurality of three-dimensional blocks with volumes less than or equal to one cubic millimeter, having the same size and randomly selected from the hierarchical porous material, are substantially the same.

12. The hierarchical porous material of claim 11, wherein the masses are substantially the same means, the plurality of three-dimensional blocks with volumes less than or equal to one cubic millimeter and having the same size, are randomly selected from the hierarchical porous material, each of the plurality of three-dimensional block is measured to obtain an average value of the mass, and an absolute value of a deviation of the mass of any three-dimensional block from the mass average value is less than or equal to 4% of the mass average value of the plurality of three-dimensional blocks.
